# EUROPÄISCHE PATENTSCHRIFT

(11) **EP 1 060 760 B1**
(45) Veröffentlichungstag und Bekanntmachung des Hinweises auf die Patenterteilung: **07.07.2004**
(21) Anmeldenummer: 00111454.5
(22) Anmeldetag: 27.05.2000
(51) Int. Cl.: A61M 39/02, A61B 17/11

(54) **Hämostatische Zugangsvorrichtung für Blutgefässe**
Hemostasis blood vessel access device
Dispositif d'accès hémostatique pour vesseaux sanguins

(30) Priorität: 16.06.1999 DE 19927422
(43) Veröffentlichungstag der Anmeldung: 20.12.2000
(73) Patentinhaber: Jostra AG, 72145 Hirrlingen (DE)
(72) Erfinder: MATHEIS, Georg Dr., D-63303 Dreieich (DE)
(74) Vertreter: Möbus, Daniela, Dr.-Ing.

(56) Entgegenhaltungen:
- US-A- 5 326 350
- US-A- 5 599 329
- US-A- 5 893 369

## Beschreibung

Bei Operationen am Herzen ist es erforderlich, das Herz vom Körper-Kreislauf abzutrennen und den Körper des Patienten über ein extrakorporales Kreislaufsystem zu versorgen. Das vom extrakorporalen Kreislaufsystem aufbereitete Blut wird über die Aorta dem Körper des Patienten wieder zugeführt. Hierzu ist eine Kanülierung der Aorta ascendens erforderlich. Die Aorta stellt jedoch ein Blutgefäß mit hohem Innendruck dar, sodass die Gefahr des Auftretens von Blutungen beim Kanülieren und Dekanülieren relativ hoch ist.

Bei Herzoperationen am geöffneten Brustkorb ist die Aorta gut zugänglich, sodass auftretende Blutungen direkt und sofort von Hand gestillt werden können. Aufgrund der wesentlich geringeren Belastung des Patienten werden heute jedoch auch in der Herzchirurgie minimal invasive und/oder Roboter assistierte Operationsverfahren bevorzugt, die möglichst ohne Eröffnung des Brustkorbs vorgenommen werden sollen. Falls es bei diesen Operationsmethoden während der Kanülierung oder Dekanülierung der Aorta zu Blutungen kommt, muss unter Umständen dennoch der Brustkorb geöffnet werden, um die lebensbedrohliche Blutung sicher stillen zu können. Das öffnen des Brustkorbs ist nicht nur ein großer Eingriff in den Körper des Patienten, sondern auch ein zeitraubender Vorgang, wodurch weitere Gefahren für die Gesundheit des Patienten entstehen können. Auch bei anderen größeren Blut führenden Gefäßen können vergleichbare Probleme auftreten.

Die Druckschrift US-A-5 893 369 zeigt eine Bypass-leitung, die aus einem Rohrkörper besteht, dessen Enden flanschartig an ein Blutgefäß augebracht werden. Während des operativen Eingrifts werden medizinische Instrumente durch den Rohrkörper eingebracht.

Der vorliegenden Erfindung liegt die Aufgabe zugrunde, eine Möglichkeit zu schaffen, Blut führende Gefäße ohne größere Eingriffe in den Körper des Patienten zu kanülieren und dekanülieren und/oder Blutungen, die beim Kanülieren oder Dekanülieren oder auch beim Punktieren und Depunktieren eines Blut führenden Gefäßes auftreten können, auf einfache Weise und ohne Risiko für die Gesundheit des Patienten bis zur vollständigen Stillung begrenzen und/oder stillen zu können.

Die Aufgabe wird mit einer Vorrichtung zur Kanülierung von Gefäßen und zur Stillung und/oder Begrenzung von Blutungen beim Kanülieren/Dekanülieren oder Punktieren/Depunktieren eines Blut führenden Gefäßes mit einem Rohrkörper gelöst, an dessen gefäßseitigem Ende ein Dichtkragen aus einem nachgiebigen Material angeordnet ist, mit dem der Rohrkörper über der einzubringenden oder schon vorhandenen Gefäßöffnung auf das Gefäß blutdicht aufsetzbar ist, wobei von der gefäßabgewandten Seite her in das Innere des Rohrkörpers Instrumente und Einrichtungen zum Kanülieren/Dekanülieren oder Punktieren/Depunktieren des Gefäßes und/oder eine hämostatische Sonde mit einer Blut dichtend an der Innenwand des Rohrkörpers anliegenden Scheibe oder Kolben zur Begrenzung von Blutungen einführbar sind. Der Rohrkörper, der vorzugsweise aus einem transparenten Material gefertigt sein kann, eröffnet eine einfache Möglichkeit, durch eine relativ kleine Körperöffnung hindurch ein Gefäß zu kanülieren und gegebenenfalls aus dem Gefäß austretendes Blut im Rohrkörper zu sammeln und mittels der hämostatischen Sonde, deren Scheibe oder Kolben vorzugsweise gasdurchlässig sein kann, den Blutaustritt zu begrenzen. Der Rohrkörper kann außerdem vorzugsweise so lang ausgeführt sein, dass er nach Aufsetzen auf das Gefäß aus dem Körper des Patienten nach außen ragt. Hierdurch können alle Kanülierungsmaßnahmen und die initialen Blutstillungsmaßnahmen vollständig außerhalb des Körpers des Patienten durchgeführt werden. Soweit die endgültige chirurgische Blutstillung einen größeren Eingriff in den Körper, beispielsweise ein Eröffnen des Brustkorbs erfordert, ist dadurch ausreichend Zeit geschaffen.

Die erfindungsgemäße Vorrichtung eignet sich daher insbesondere zum Kanülieren und Dekanülieren der Aorta bei minimal invasiven und/oder Roboter assistierten Herzoperationen. Es ist nur ein sehr kurzer Schnitt in den Brustkorb des Patienten erforderlich, durch den dann der Rohrkörper zur Aorta führbar ist. Durch den Rohrkörper kann anschließend beispielsweise ein ringförmiger Applikator für chirurgische Clips zur Erzeugung einer Tabaksbeutelnaht zum Gefäß führbar sein, wobei die Clips auf dem Umfang des Applikators verteilt angeordnet sind und durch die Clips ein Faden für die Tabaksbeutelnaht gezogen ist. Mit der Tabaksbeutelnaht wird nach dem Legen der Kanüle eine blutdichte Verbindung zwischen der Kanüle und dem Gefäß, beispielsweise der Aorta, erzeugt. Außerdem wird mit der Tabaksbeutelnaht nach dem Dekanülieren die öffnung in der Gefäßwand wieder verschlossen. Die Enden des Fadens der Tabaksbeutelnaht können entweder durch den Rohrkörper oder durch am Umfang des Rohrkörpers vorgesehene Lumen nach außen geführt sein, sodass das Verengen der Gefäßöffnung durch Ziehen an den beiden Fadenenden ebenfalls vollständig außerhalb des Körpers des Patienten erfolgen kann.

In den Rohrkörper kann außerdem eine Führungsscheibe für ein Skalpell oder ein Messer, die die Bewegung des Skalpells oder Messers in axialer und radialer Richtung des Rohrkörpers begrenzt, einführbar sein. Der Einstich in das Gefäß erfolgt also geführt, sodass der Einschnitt weder zu tief noch zu lang erfolgen kann. Falls es dennoch zu Blutungen aus dem Gefäß kommen sollte, kann dies in dem Rohrkörper aufgefangen und entweder durch die Führungsscheibe selbst oder durch eine hämostatische Sonde gestoppt werden.

über die Führungsscheibe und das Skalpell oder Messer kann außerdem eine Kanüle durch den Rohrkörper zur Gefäßöffnung führbar sein. Hierdurch ist es möglich, beim Zurückziehen des Skalpells gleichzeitig die Kanüle nach vorne zu schieben und mit ihr die den vom Skalpell ausgeführten Schnitt in die Gefäßwand aufzuweiten. An die Kanüle kann anschließend eine Schlauchleitung eines extrakorporalen Kreislaufsystems, beispielsweise einer Herz-Lungen-Maschine angeschlossen werden. Während des gesamten Kanülierungsvorgangs sorgt der Rohrkörper der erfindungsgemäßen Vorrichtung dafür, dass eventuell aus dem Gefäß austretendes Blut nicht in den Körper des Patienten austritt, sondern im Rohrkörper selbst aufgefangen und dadurch die Blutung begrenzt werden kann.

Auch während des Dekanülierens des Blutgefäßes leistet die erfindungsgemäße Vorrichtung diese Sicherheitsfunktion. Zum Dekanülieren kann durch das Innere der Kanüle ein Okluder mit einer konischen Spitze in das Gefäß einführbar sein, der die Gefäßöffnung nach dem Herausziehen der Kanüle abdichtet. Anschließend kann auch dieser Okluder langsam aus der Gefäßöffnung herausgezogen werden, während die Tabaksbeutelnaht durch Ziehen an den Fadenenden sukzessive geschlossen wird, bis die Spitze des Okluders vollständig aus der Gefäßöffnung herausgezogen ist. Durch diese Art der Dekanülierung ist der Blutaustritt aus dem Gefäß nach dem Zurückziehen der Kanüle nur minimal. Sollte es dennoch zu stärkeren Blutungen kommen, so kann dieses wieder im Rohrkörper aufgefangen und bis zur chirurgischen Blutstillung begrenzt werden.

An der Spitze des Okluders kann auch ein gefaltetes oder deformierbares plattenförmiges Element angeordnet sein, das sich nach Einführen in das Gefäß entfaltet und beim Zurückziehen des Okluders aus dem Gefäß die Gefäßöffnung von innen verschließt und das auf der Gefäßaußenseite mit einem Verankerungselement, vorzugseise einer Gegenplatte, verbindbar ist. Diese Art des Verschlusses der Gefäßöffnung kann die üblicherweise eingesetzten Tabaksbeutelnähte ersetzen und zeichnet sich dadurch aus, dass sie rasch und einfach zu platzieren ist.

Bei der Kanülierung und Dekanülierung der Aorta durch den geschlossenen Brustkorb hindurch ist mit der erfindungsgemäßen Vorrichtung beim Auftreten von Blutungen aus der Aorta ein öffnen des Brustkorbs zur Stillung der Blutung nicht mehr erforderlich.

Die erfindungsgemäße Vorrichtung eignet sich jedoch nicht nur für den Einsatz bei der Aorta, sondern auch bei anderen Blut führenden Gefäßen.

Nachfolgend werden bevorzugte Ausführungsbeispiele einer erfindungsgemäßen Vorrichtung zur Gefäßkanülierung und Begrenzung bzw. Stillung von Blutungen anhand der Zeichnung näher beschrieben.

Es zeigen:
- Fig. 1: eine schematische Ansicht einer auf einem Gefäß platzierten erfindungsgemäßen Vorrichtung;
- Fig. 2: eine schematische Ansicht des Einführens eines Nahtlegegeräts in den Rohrkörper der Vorrichtung aus Fig. 1;
- Fig. 3: eine schematische Ansicht des Einführens einer Führungsscheibe eines Skalpells in den Rohrkörper der Vorrichtung nach Fig. 1;
- Fig. 4: eine schematische Ansicht des Einführens einer Kanüle durch den Rohrkörper aus Fig. 1;
- Fig. 5: eine schematische Detailansicht eines Rohrkörpers einer Vorrichtung nach Fig. 1 mit Kanüle und Okluder.

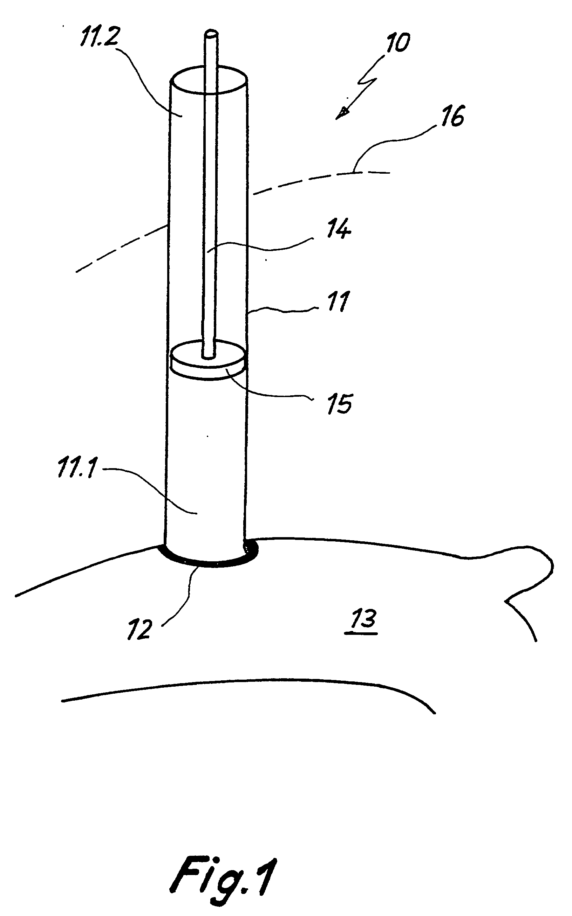

Fig. 1 zeigt eine Vorrichtung 10 zur Stillung von Blutungen gemäß der Erfindung mit einem Rohrkörper 11, der an seinem gefäßseitigen Ende 11.1 einen Dichtkragen 12 aus einem weichen Material aufweist, mit dem der Rohrkörper 11 blutdicht auf ein Blutgefäß, hier eine Aorta 13, aufsetzbar ist. Von der dem Gefäß 13 abgewandten Seite 11.2 des Rohrkörpers 11 her, kann eine hämostatische Sonde 14 mit einer den Querschnitt des Rohrkörpers 11 blutdicht verschließenden Scheibe 15 eingeführt werden. Der Rohrkörper 11 ist aus einem transparenten Material gefertigt, sodass die Position der Sonde 14 gut zu kontrollieren ist. Der Rohrkörper 11 ragt über die gestrichelt eingezeichnete Körperoberfläche 16 des Patienten nach außen, sodass sämtliche Manipulationen am und im Rohrkörper 11 vom Körperäußeren her erfolgen können.

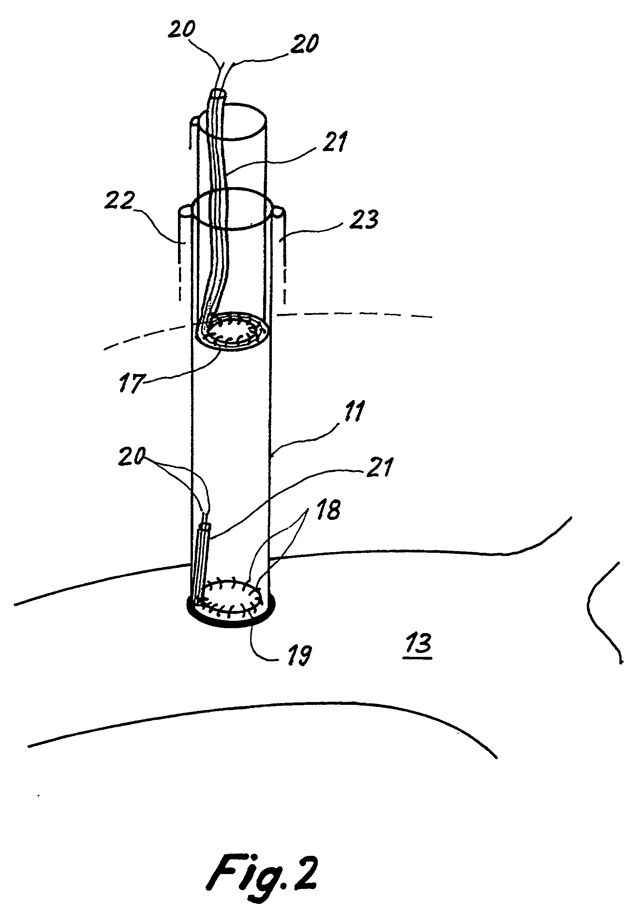

In Fig. 2 ist das Einführen eines Applikators 17 für kreisförmig angeordnete chirurgische Clips 18 durch den Rohrkörper 11 dargestellt. Durch die Clips 18 ist ein Faden 19 gezogen, der nach dem Einsetzen der Clips in die Wand der Aorta 13 eine Tabaksbeutelnaht bildet. Die Enden 20 des Fadens 19 sind durch eine Hülse 21 gezogen und werden aus dem Rohrkörper 11 nach außen geleitet. Der Rohrkörper 11 kann jedoch auch auf seiner Außenseite mit zwei in der Figur lediglich angedeuteten Lumen 22 und 23 für die Fadenenden 20 versehen sein, sodass das Herausführen der Fadenenden 20 den inneren Querschnitt des Rohrkörpers 11 nicht verengt.

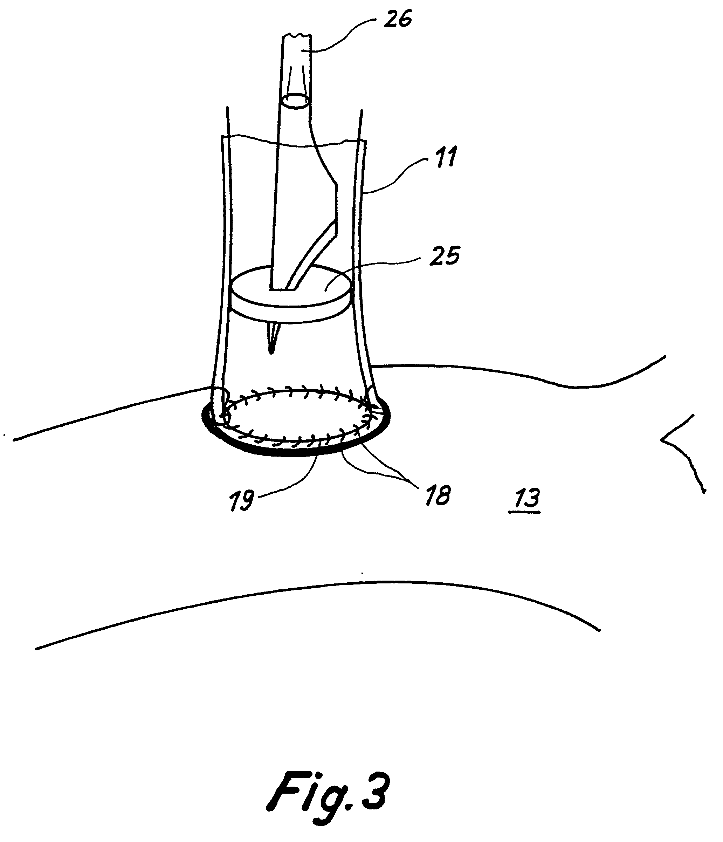

Nach der Herstellung der Tabaksbeutelnaht kann in das Innere des Rohrkörpers 11 eine Führungsscheibe 25 für ein Skalpell 26 eingeführt werden, wie in Fig. 3 gezeigt ist. Die Scheibe 25 kann dabei so ausgebildet sein, dass sie die Bewegung des Skalpells sowohl in Längsrichtung als auch in radialer Richtung des Rohrkörpers 11 begrenzt. Dadurch kann verhindert werden, dass der durch das Skalpell 26 in die Gefäßwand eingebrachte Schnitt zu lang wird oder, dass das Skalpell 26 zu tief in das Gefäß eindringt und gegebenenfalls die gegenüberliegende Gefäßwand verletzt. Gleichzeitig bildet die Führungsscheibe 25 eine Dichtung für gegebenenfalls aus dem Schnitt nach außen strömendes Blut.

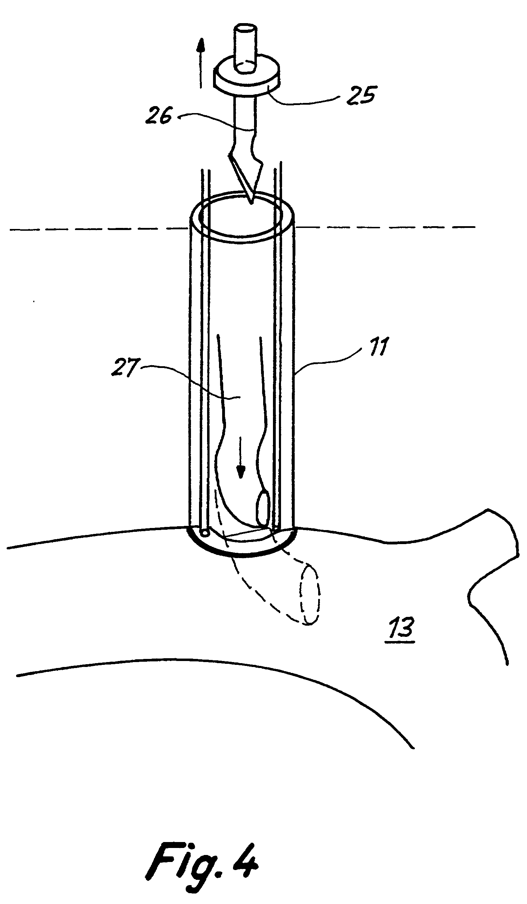

Nach dem Setzen des Schnitts in das Gefäß 13 wird das Skalpell 26 mit der Führungsscheibe 25 aus dem Rohrkörper 11 nach außen gezogen und gleichzeitig eine Kanüle 27 durch den Rohrkörper 11 nach unten und durch die Gefäßöffnung hindurchgeführt, wie Fig. 4 zeigt. Die Kanüle 27 weitet dabei den Schnitt auf, sodass die Gefäßwand die Kanüle 27 eng umschließt. Zusätzlich kann jedoch auch die Tabaksbeutelnaht zusammengezogen und dadurch der Gefäßöffnungsrand dicht an die Kanüle 27 angelegt werden. Das nach wie vor auf dem Gefäß 13 befindliche Rohr 11 dient dabei als Sicherheitseinrichtung für gegebenenfalls dennoch aus der Gefäßöffnung austretendes Blut.

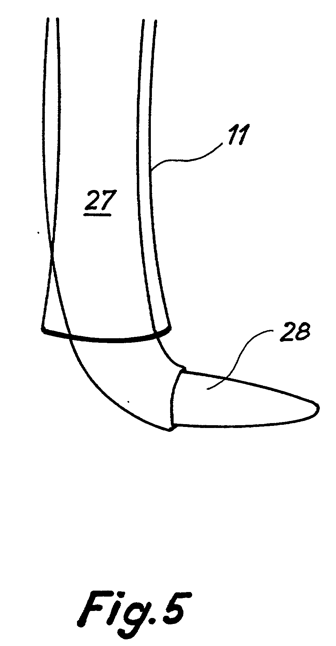

Wie Fig. 5 zeigt, erfüllt der Rohrkörper 11 diese Sicherheitsfunktion auch beim Dekanülieren des Gefäßes 13. Hierzu wird in die Kanüle 27 ein Okluder 28 mit einer konischen Spitze eingeführt. Zunächst wird die Kanüle 27 aus dem Gefäß 13 zurückgezogen und anschließend der Okluder 28, wobei die Tabaksbeutelnaht sukzessive zusammengezogen wird, bis die konische Spitze des Okluders 28 vollständig aus der Gefäßöffnung zurückgezogen und die Gefäßöffnung ganz geschlossen ist.

## Patentansprüche

1. Kanülierungsvorrichtung für Gefäße zur Stillung und/oder Begrenzung von Blutungen beim Kanülieren/Dekanülieren oder Punktieren/Depunktieren eines Blut führenden Gefäßes (13) mit einem aus einem tranparenten Material gefertigten Rohrkörper (11), an dessen gefäßseitigem Ende (11.1) ein Dichtkragen (12) aus einem nachgiebigen Material angeordnet ist, mit dem der Rohrkörper (11) über der einzubringenden oder schon vorhandenen Gefäßöffnung auf das Gefäß (13) blutdicht aufsetzbar ist, wobei von der gefäßabgewandten Seite (11.2) her in das Innere des Rohrkörpers (11) Instrumente (26) und Einrichtungen (17, 27, 28) zum Kanülieren/Dekanülieren oder Punktieren/Depunktieren des Gefäßes (13) und/oder eine hämostatische Sonde (14) mit einer Blut dichtend an der Innenwand des Rohrkörpers (11) anliegenden Scheibe (15) oder Kolben zur Begrenzung von Blutungen einführbar sind.

2. Vorrichtung nach Anspruch 1, **dadurch gekennzeichnet, dass** der Rohrkörper (11) eine derartige Länge im Verhältnis zu den Maßen des Körpers eines Patienten aufweist, dass er nach Aufsetzen auf das Gefäß (13) aus dem Körper des Patienten nach außen ragt.

3. Vorrichtung nach einem der Ansprüche 1 bis 2, **dadurch gekennzeichnet, dass** die Scheibe (15) oder der Kolben der hämostatischen Sonde (14) gasdurchlässig ist.

4. Vorrichtung nach einem der Ansprüche 1 bis 3, **dadurch gekennzeichnet, dass** ein ringförmiger Applikator (17) für chirurgische Clipse (18) vorgesehen ist, der durch den Rohrksirper (11) zur Erzeugung einer Tabaksbeutelnaht zum Gefäß (13) führbar ist, wobei die Clipse (18) auf dem Umfang des Applikators (17) verteilt angeordnet sind und durch die Clipse (18) ein Faden (19) für die Tabaksbeutelnaht gezogen ist.

5. Vorrichtung nach Anspruch 4, **dadurch gekennzeichnet, dass** am Umfang des Rohrkörpers (11) Lumen (22, 23) vorgesehen sind, durch die die Enden (20) des Fadens (19) nach außen geführt sind.

6. Vorrichtung nach einem der Ansprüche 1 bis 5, **dadurch gekennzeichnet, dass** eine Führungsscheibe (25) für ein Skalpell (26) oder ein Messer vorgesehen sind, die die Bewegung des Skalpells (26) oder des Messers in axialer und radialer Richtung des Rohrkörpers (11) begrenzen und die in den Rohrkörper (11) einführbar sind.

7. Vorrichtung nach Anspruch 6, **dadurch gekennzeichnet, dass** über die Führungsscheibe (25) und das Skalpell (26) oder Messer eine Kanüle (27) durch den Rohrkörper (11) zur Gefäßöffnung führbar ist.

8. Vorrichtung nach einem der Ansprüche 1 bis 7, **dadurch gekennzeichnet, dass** ein Okluder (28) mit einer konischen Spitze vorgesehen ist, der zum Dekanülieren des Gefäßes durch das Innere der Kanüle (27) in das Gefäß (13) einführbar ist, wobei der Okluder (28) die Kanüle (27) abdichtet.

9. Vorrichtung nach Anspruch 8, **dadurch gekennzeichnet, dass** an der Spitze des Okluders (28) ein plattenförmiges Element angeordnet ist, das beim Zurückziehen des Okluders (28) aus dem Gefäß (13) die Gefäßöffnung von innen verschließt und das auf der Gefäßaußenseite mit einem Verankerungselement, vorzugsweise einer Gegenplatte, verbindbar ist.

## Claims

1. A cannulation apparatus for blood vessels for stopping and/or limiting bleeding during the cannulation/decannulation or aspiration/deaspiration of a blood-carrying vessel (13), comprising a tubular body (11) made of a transparent material, at the vessel end (11.1) of which a sealing collar (12) is disposed which is made of a flexible material and with which the tubular body (11) can be placed, forming a blood-tight seal, on the vessel (13) over the vessel opening which is to be introduced or which is already present, wherein instruments (26) and devices (17, 27, 28) for the cannulation/decannulation or aspiration/deaspiration of the vessel (13) and/or a haemostatic probe (14) can be introduced from the end (11.2) remote from the vessel into the interior of the tubular body (11), with a disc (15) or piston which is impervious to blood and which is seated against the inner wall of the tubular body (11) in order to limit bleeding.

2. An apparatus according to claim 1, **characterised in that** in relation to the dimensions of the body of a patient the tubular body (11) has a length such that after placement on the vessel (13) it protrudes outwards from the body of the patient.

3. An apparatus according to either one of claims 1 and 2, **characterised in that** the disc (15) or piston of the haemostatic probe (14) is permeable to gases.

4. An apparatus according to any one of claims 1 to 3, **characterised in that** an annular applicator (17) for surgical clips (18) is provided and can be passed through the tubular body (11) to the vessel (13) in order to produce a purse-string suture, wherein the clips (18) are disposed distributed on the periphery of the applicator (17) and a thread (19) for the purse-string suture is pulled through the clips (18).

5. An apparatus according to claim 4, **characterised in that** lumens (22, 23) through which the ends (20) of the thread (19) are led outwards are provided at the periphery of the tubular body (11).

6. An apparatus according to any one of claims 1 to 5, **characterised in that** a guide disc (25) for a scalpel (26) or knife is provided, which limits the movement of the scalpel (26) or knife in the axial and radial directions of the tubular body (11) and which can be introduced into the tubular body (11).

7. An apparatus according to claim 6, **characterised in that** a cannula (27) can be passed through the tubular body (11) to the vessel opening via the guide disc (25) and the scalpel (26) or knife.

8. An apparatus according to any one of claims 1 to 7, **characterised in that** an occluder (28) with a conical tip is provided for the decannulation of the vessel and can be introduced into the vessel (13) through the interior of the cannula (27), wherein the occluder (28) seals the cannula (27).

9. An apparatus according to claim 8, **characterised in that** a plate-shaped element is disposed at the tip of the occluder (28), which plate-shaped element closes the vessel opening from the inside when the occluder (28) is withdrawn from the vessel (13) and which can be joined to an anchoring element, preferably a counter-plate, on the outside of the vessel.

## Revendications

1. Dispositif de pose d'une canule pour vaisseaux afin de stopper et/ou de limiter des saignements lors de la pose/dépose d'une canule ou lors de l'introduction/extraction d'une aiguille à ponctionner un vaisseau (13) conduisant le sang, comportant un corps tubulaire (11) réalisé dans une matière transparente, à l'extrémité (11.1) côté vaisseau duquel est disposée une collerette d'étanchéité (12) en un matériau souple par lequel le corps tubulaire (11) peut être placé de manière étanche au sang sur le vaisseau (13), par l'ouverture de vaisseau à pratiquer ou déjà existante, des instruments (26) et dispositifs (17, 27, 28) pour pose/dépose d'une canule ou d'une aiguille à ponctionner le vaisseau (13) et/ou une sonde hémostatique (14) avec un disque (15) ou piston s'appliquant de manière étanche au sang contre la paroi intérieure du corps tubulaire (11), peuvent être introduits depuis le côté (11.2) opposé au vaisseau, à l'intérieur du corps tubulaire (11), afin de limiter les saignements.

2. Dispositif selon la revendication 1, **caractérisé en ce que** le corps tubulaire (11) présente une longueur telle par rapport aux dimensions du corps d'un patient, qu'après mise en place sur le vaisseau (13), il dépasse vers l'extérieur du corps du patient.

3. Dispositif selon l'une des revendications 1 à 2, **caractérisé en ce que** le disque (15) ou le piston de la sonde hémostatique (14) est perméable aux gaz.

4. Dispositif selon l'une des revendications 1 à 3, **caractérisé en ce qu'**il est prévu un applicateur (17) de forme annulaire pour clips chirurgicaux (18), qui peut être guidé à travers le corps tubulaire (11) pour produire une couture en aumonière par rapport au vaisseau (13), les clips (18) étant agencés d'une manière répartie sur le pourtour de l'applicateur (17) et un fil (19) étant tiré à travers les clips (18) pour la couture en aumonière.

5. Dispositif selon la revendication 4, **caractérisé en ce que** sur le pourtour du corps tubulaire (11) sont prévues des ouvertures (22, 23) à travers lesquelles les extrémités (20) du fil (19) sont guidées vers l'extérieur.

6. Dispositif selon l'une des revendications 1 à 5, **caractérisé en ce qu'**est prévu un disque de guidage (25) pour un scalpel (26) ou une lame qui limite le mouvement du scalpel (26) ou de la lame, dans la direction axiale et la direction radiale du corps tubulaire (11), et qui peut être introduit dans le corps tubulaire (11).

7. Dispositif selon la revendication 6, **caractérisé en ce que** par le disque de guidage (25) et le scalpel (26) ou la lame on peut guider une canule (27) à travers le corps tubulaire (11) vers l'ouverture du vaisseau.

8. Dispositif selon l'une des revendications 1 à 7, **caractérisé en ce qu'**il est prévu un dispositif d'occlusion (28) avec une pointe conique qui, pour extraire la canule du vaisseau, peut être introduit à travers l'intérieur de la canule (27), dans le vaisseau (13), le dispositif d'occlusion (28) assurant l'étanchéité de la canule (27).

9. Dispositif selon la revendication 8, **caractérisé en ce qu'**à la pointe du dispositif d'occlusion (28) est disposé un élément en forme de plaque qui, lorsque le dispositif d'occlusion (28) est retiré du vaisseau (13), ferme l'ouverture du vaisseau depuis l'intérieur et qui peut être relié, sur le côté extérieur du vaisseau, à un élément d'ancrage, de préférence une contreplaque.
